(19) Europäisches Patentamt / European Patent Office / Office européen des brevets

(11) **EP 3 402 457 B1**

(12) **EUROPEAN PATENT SPECIFICATION**

(45) Date of publication and mention
of the grant of the patent:
**25.02.2026 Bulletin 2026/09**

(21) Application number: **17738735.4**

(22) Date of filing: **16.01.2017**

(51) International Patent Classification (IPC):
*A61K 9/107* $^{(2006.01)}$    *A61K 47/42* $^{(2017.01)}$
*A61K 8/06* $^{(2006.01)}$     *A23L 27/00* $^{(2016.01)}$
*A23L 33/10* $^{(2016.01)}$

(52) Cooperative Patent Classification (CPC):
**A61K 47/42; A23L 27/70; A23L 33/10;
A61K 8/0279; A61K 8/062; A61K 8/64;
A61K 9/1075; A61K 47/44; A61Q 19/00;**
A61K 2800/10; A61K 2800/33; A61K 2800/412;
A61K 2800/56

(86) International application number:
**PCT/SG2017/050020**

(87) International publication number:
**WO 2017/123160 (20.07.2017 Gazette 2017/29)**

(54) **PROTEIN CAGE-STABILIZED PICKERING EMULSIONS AND THE USE THEREOF**

PROTEINCAGE-STABILISIERTE PICKERING-EMULSIONEN UND VERWENDUNG DAVON

ÉMULSIONS DE PICKERING STABILISÉES PAR UNE CAGE PROTÉIQUE ET UTILISATION DE CES DERNIÈRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.01.2016 SG 10201600290W**

(43) Date of publication of application:
**21.11.2018 Bulletin 2018/47**

(73) Proprietors:
• **Nanyang Technological University**
**Singapore 639798 (SG)**
• **Agency for Science, Technology and Research**
**Singapore 138632 (SG)**

(72) Inventors:
• **LIM, Sierin**
**Singapore 639798 (SG)**
• **SARKER, Mridul**
**Singapore 639798 (SG)**
• **TOMCZAK, Nikodem**
**Singapore 138632 (SG)**

(74) Representative: **Viering, Jentschura & Partner
mbB
Patent- und Rechtsanwälte
Hamborner Straße 53
40472 Düsseldorf (DE)**

(56) References cited:
**WO-A1-2014/090920     WO-A1-2015/192603**

• **Supporting technical information: Distribution of
Charges in Protein cages - Emulsification
characteristics**
• **Supplementary experimental evidence:
Pickering Emulsion using AfFtn-AA (29 Oct 2021)**
• **JOHNSON E ET AL: "Crystal Structures of a
Tetrahedral Open Pore Ferritin from the
Hyperthermophilic Archaeon Archaeoglobus
fulgidus", STRUCTURE, ELSEVIER,
AMSTERDAM, NL, vol. 13, no. 4, 1 April 2005
(2005-04-01), pages 637 - 648, XP027638594,
ISSN: 0969-2126, [retrieved on 20050401]**
• **Supplementary experiment data Exhibit A
(19.5.2023)**
• **Declaration Sierin Lim, Ph.D., 28-11-2023**

**(Cont. next page)**

- VAN RIJN P. ET AL.: "Pickering emulsion templated soft capsules by self- assembling cross-linkable ferritin-polymer conjugates", CHEM. COMMUN., vol. 47, no. 29, 20 June 2011 (2011-06-20), pages 8376 - 8378, XP055399350, [retrieved on 20170222]
- JUTZ G. ET AL.: "Bio-inorganic microcapsules from templating protein- and bionanoparticle-stabilized Pickering emulsions", J. MATER. CHEM., vol. 20, no. 21, 23 March 2010 (2010-03-23), pages 4299 - 4304, XP055142317, [retrieved on 20170222]
- FUJII S. ET AL.: "Ferritin as a bionano-particulate emulsifier", J. COLLOID INTERFACE SCI., vol. 388, no. 1, 17 June 2009 (2009-06-17), pages 222 - 228, XP027567037, [retrieved on 20170222]
- RUSSE L J. T. ET AL.: "Selt-Assembly and Cross-Linking ot Bionanoparticles at Liquid-Liquid Interfaces", ANGEW CHERN.LNT. ED., vol. 44, no. 16, 1 April 2005 (2005-04-01), pages 2420 - 2426, XP055399351, [retrieved on 20170222]
- KAUR G. ET AL.: "Interfacial Assembly of Turnip Yellow Mosaic Virus Nanoparticles", LANGMUIR, vol. 25, no. 9, 4 August 2009 (2009-08-04), pages 5168 - 5176, XP055399353, [retrieved on 20170223]
- LIM S. ET AL.: "Protein Cages as Theranostic Agent Carriers", WORLD CONGRESS ON MEDICAL PHYSICS AND BIOMEDICAL ENGINEERING - IFMBE PROCEEDINGS, vol. 39, 26 May 2012 (2012-05-26), pages 321 - 324, XP008179531, [retrieved on 20170222]
- REN D. ET AL.: "Engineered drug-protein nanoparticle complexes forfolate receptor targeting", BIOCHEM. ENG. J., vol. 89, 24 September 2013 (2013-09-24), pages 33 - 41, XP028861676, [retrieved on 20170223]
- LAM S. ET AL.: "Pickering stabilization of foams and emulsionswith particles of Biological origin", CURR. OPIN. COLLOID INTERFACE SCI., vol. 19, no. 5, 30 July 2014 (2014-07-30), pages 490 - 500, XP055291601, [retrieved on 20170223]

Remarks:
•The complete document including Reference Table(s) and the Sequence Listing(s) can be downloaded from the EPO website
•The file contains technical information submitted after the application was filed and not included in this specification

**Description**

This application makes reference to and claims the benefit of priority of the Singapore Patent Application No. 10201600290W filed on 14 January 2016.

**FIELD OF THE INVENTION**

The present invention relates generally to Pickering emulsions stabilized by protein cages.

**BACKGROUND OF THE INVENTION**

Pickering emulsions were first reported by Pickering (Pickering, Journal of the Chemical Society, Transactions 1907, 91 (0), 2001-2021) and Ramsden (Ramsden, Proceedings of the Royal Society of London 1903, 72 (477-486), 156-164). They are formed by self-assembly of colloidal particles at the interface of two immiscible liquid phases as favored by decreased adsorption free energy. Adsorption of colloidal particles is irreversible, as the energy of desorption is too high, making the emulsion very stable. Varieties of inorganic particles such as silica, clay, latex, and Au nanoparticles have been used as Pickering emulsion stabilizers because of their well-defined shapes and narrow size distribution. However, such inorganic particles are not suitable for applications requiring biocompatibility and biodegradability.

In recent years, particles of biological origin, such as cellulose, chitosan, lignin, modified starch, flavonoids, lipid nanoparticles, water soluble zein protein, soy protein, casein micelles as well as microorganism e.g. viruses and bacteria cells, have been employed to stabilize Pickering emulsions. In addition, different viruses, like tobacco mosaic virus, cowpea mosaic virus, turnip yellow mosaic virus have been used as nano-scale particulate emulsifiers to stabilize oil-in-water emulsions.

However, the use of these biomolecules as Pickering emulsifiers suffers from their polydispersity and requirement for additional additives for optimal surface activities. In addition, the use of viral particles is also limited in food, cosmetic, and pharmaceutical fields. The colloidal and wetting properties of the bionanoparticles are also poorly characterized for complex food system.

Fujii S. et al., Journal of Colloid and Interface Science 2009, 388(1), 222-228, discloses stable 'Pickering-type' emulsions prepared using ferritin.

Therefore, there is still need in the art for alternative nanoparticles with high surface activity and wide range of compatibility for use as emulsifiers in Pickering emulsions.

**SUMMARY OF THE INVENTION**

The present invention is directed to meet the aforementioned need in the art, and provides protein cages for use as Pickering emulsifiers.

In a first aspect, the present invention therefore relates to a Pickering emulsion comprising an aqueous phase, an oil phase immiscible with said aqueous phase, and a nanoparticle dispersed in said aqueous phase and adsorbed to the liquid-liquid interface between said aqueous phase and said oil phase, wherein said nanoparticle is a protein cage, wherein the protein cage is composed of protein units selected from the group consisting of *Bacillus stearothermophilus* E2 protein of pyruvate dehydrogenase multi-enzyme complex (E2) having the amino acid sequence of SEQ ID NO:1, E2LC2 protein having the amino acid sequence of SEQ ID NO:2.

In various embodiments, the aqueous phase is any one selected from the group consisting of water and aqueous solutions.

In various embodiments, the oil is selected from the group consisting of essential oils, including but not limited to rosemary oil, vegetable oils, mineral oils, organic oils, and lipids.

In certain embodiments, the oil is rosemary oil and the protein cage is composed of protein units of *Bacillus stearothermophilus* E2 protein of pyruvate dehydrogenase multi-enzyme complex (E2) having the amino acid sequence of SEQ ID NO:1 or E2LC2 protein having the amino acid sequence of SEQ ID NO:2.

In various embodiments, the aqueous phase comprises a first agent.

In various embodiments, the oil phase comprises a second agent.

In various embodiments, the protein cage is coupled to or loaded with a third agent.

In various embodiments, the first, second, or third agent is selected from a protein (different from those forming the protein cage), nucleic acid molecule, organic compound, inorganic compound, or any other molecule.

In various embodiments, the first, second, or third agent is selected from a therapeutic agent, a nutritional or nutraceutical agent, and a cosmetic ingredient.

In various embodiments, the Pickering emulsion is a controlled delivery system for the first, second, or third agent.

In another aspect, the present invention relates to use of the presently disclosed Pickering emulsion in pharmaceutical, cosmetic, or food applications.

In a third aspect, the present invention relates to use of the presently disclosed Pickering emulsion as a controlled release delivery system.

In a final aspect, the present invention relates to use of the protein cages disclosed herein as emulsifiers in Pickering emulsions, such as those disclosed herein.

## BRIEF DESCRIPTION OF THE DRAWINGS

The invention will be better understood with reference to the detailed description when considered in conjunction with the non-limiting examples and the accompanying drawings.

Figure 1: (A) 3-fold crystallographic representation of an E2LC2 protein cage, which consists of 60 identical subunits, twelve 5-nm openings, and is of 24 nm in outer diameter. Sites for conjugation of molecules are highlighted in red (aspartic acid, amino acid #381) and blue (Glycine, amino acid#382) (B) Transmission electron microscope (TEM) image of dodecahedron hollow cage shape of E2LC2. The scale bar represents 50 nm. (C) Microstructure of coarse emulsion; oil phase has been stained by Nile red and E2LC2 protein cages are conjugated with Aleaflour488. The scale bar represents 5 μm.

Figure 2: The effect of oil/water ratio and protein mass fraction on (A) Emulsion Stability Index (ESI) and (B) droplet size profiling of Pickering emulsion after 10 days of shelf-life at ambient condition. Inset is the zoom-in of the area marked with red rectangle.

Figure 3: The effect of pH on (A) zeta potential and (B) droplet size of dispersed phase of freshly prepared Pickering emulsion with 0.35% protein mass fraction and oil/water ratio 0.11. The error bar represents the standard error in measurement.

Figure 4: Droplet size at pH 4 at the end of each stabilization and destabilization cycle.

Figure 5: (A, B) The effect of ionic concentrations on zeta potential and droplet size of Pickering emulsion at different storage length; (C, D) The effect of storage temperature on zeta potential and droplet size of Pickering emulsion at different storage periods. Pickering emulsion with 0.35% E2LC2 mass fraction and rosemary oil/water ratio 0.11 at pH 8.7 was used to conduct this investigation. Inset in A is the image of the samples at day 2. The error bar represents the standard error in measurement.

Figure 6.1: Rheological analysis of (A) emulsion (oil/water ratio (owr) = 0.11) and (B) emulsion with gel-like network (owr = 0.66) by flow analysis plotted as shear stress vs. shear rate; Figure 6.2: Frequency sweep analysis plotted as loss and storage modulus vs. angular frequency; (A) with oil-water ratio (v/v) =0.11 and (B) with oil-water ratio (v/v) =0.66.

Figure 7: (A) Image of emulsion formulated at rosemary oil/water ratio of 0.66 (v/v) and E2LC2 protein mass fraction of 0.35% (wt %); (B) Electron micrograph of the emulsion gel-like system.

Figure 8: (A) SDS-PAGE analysis of E2LC2 protein cage. (B) Zeta potential of E2LC2 protein cage at different pH values.

Figure 9: (A) The visual representation of E2LC2-stabilized Pickering emulsion shows the immiscibility of the emulsion with rosemary oil phase (A) and miscibility of the emulsion with water phase (B).

Figure 10: Surface coverage (%) of rosemary oil droplet by E2LC2 protein cage at different mass fraction of E2LC2 (wt%) at constant oil/water ratio of 0.11.

Figure 11: A schematic representation of the Pickering emulsion for use as an agent delivery system.

## DETAILED DESCRIPTION OF THE INVENTION

The following detailed description refers to, by way of illustration, specific details and embodiments in which the invention may be practiced. These embodiments are described in sufficient detail to enable those skilled in the art to practice the invention. The various embodiments are not necessarily mutually exclusive, as some embodiments can be combined with one or more other embodiments to form new embodiments.

Unless otherwise defined, all technical and scientific terms used herein have the same meaning as commonly understood by one of ordinary skill in the art. In case of conflict, the present document, including definitions, will control.

In a first aspect, the present invention relates to a Pickering emulsion comprising an aqueous phase, an oil phase immiscible with said aqueous phase, and a nanoparticle dispersed in said aqueous phase and adsorbed at any orientation and contact angle to the liquid-liquid interface between said aqueous phase and said oil phase, wherein said nanoparticle is a protein cage as defined in claim 1.

The articles "a" and "an" are used herein to refer to one or to more than one (i.e., to at least one) of the grammatical object of the article. By way of example, "an element" means at least one element and can include more than one element.

The term "emulsion" as used herein refers to a preparation of a first liquid (dispersed phase) dispersed, for example in the form of drops or droplets, in a second liquid immiscible with the first one (continuous phase). In preferred embodiments, the emulsion of the present invention is an oil-in-water emulsion, i.e. the dispersed phase is an oil and the continuous phase is an aqueous phase. Alternatively, the emulsion may be a water-in-oil emulsion, i.e. the dispersed phase is an aqueous phase and the continuous phase is an oil. The emulsions described herein are liquid at room temperature (20°C) and standard pressure (1013 mbar).

Also encompassed in the term "emulsion" are emulsion-gels having a viscosity high enough to make the emulsion a gel at room temperature and standard pressure. The term "gel" as used herein means any solid or semi-solid gelled material. The physico-chemical differences between regular liquid Pickering emulsions and Pickering emulsion-gels are attributable to the compositions of the oil phase, the water phase, and the mass fraction of the protein cages.

Unlike traditional emulsions using surfactants as stabilizers, Pickering emulsions are emulsion systems in which traditional surfactants are replaced by particles. The mechanism of stabilizing the emulsions mainly involves absorbing particles to the oil-water interface to form a single-layered or multi-layered structure of particles (around the dispersed phase droplets) to stabilize the emulsion. Compared with traditional emulsions containing surfactants, Pickering emulsions have very high emulsion stability. Without wishing to be bound to any particular theory, one possible explanation for such strong adherence of particles to the fluid-fluid interfaces is that the particles are partly wettable by the two phases, with the depth of the surface energy being a function of temperature, particle size, and surface tension.

In various embodiments, the aqueous phase in accordance with the present invention is any one selected from the group consisting of water and aqueous solutions that contain, for example, salts, such as buffering salts, including phosphate, citrate and Tris, and/or water-miscible organic solvents, such as alcohols, including for example glycerol and 1,2-propane diol. Specific examples include, but are not limited to purified water, water for injection, aqueous glycerol solution, ionic liquids, aqueous solutions of buffering salts or clinically usable transfusions. Preferably, it is any one selected from the group consisting of water for injection, phosphate buffering solution, citrate buffering solution and Tris (Tris(hydroxymethyl)aminomethane) buffering solution, or the combination of at least two selected therefrom. Preferably, said phosphate buffering solution, citrate buffering solution or Tris buffering solution independently has a pH value of 5.0-10.0.

In various embodiments, the oil is an oil that is liquid at room temperature (20°C; 1013mbar). It may, for example, be selected from the group consisting of essential oils, vegetable oils, mineral oils, organic oils, lipids, and any water-immiscible liquids. The oil may comprise, consist essentially of or consist of fatty acids and/or glycerides, such as triglycerides. In some embodiments, the oil is metabolizable by living organisms, particularly mammals, such as humans. The exemplary metabolizable oil may be any plant oil, fish oil, animal oil or synthetic oil which is non-toxic to recipients and transformable by metabolism. Such oils include, but are not limited to rosemary oil, soybean oil, midchain oil, fish oil, vitamin E, vitamin E succinate, vitamin E acetate, safflower oil, corn oil, sea buckthron oil, linseed oil, peanut oil, tea-seed oil, sunflower seed oil, apricot kernel oil, coix seed oil, evening primrose seed oil, sesame oil, cottonseed oil, castor oil, low-erucic acid rapeseed oil, ethyl oleate, oleic acid, ethyl linoleate, isopropyl laurate, isopropyl myristate, ethyl butyrate, ethyl lactate, caprylic triglyceride or capric triglyceride, or the combination of at least two selected therefrom. In other embodiments, the oil is non-metabolizable by living organisms. Suitable non-limiting examples of non-metabolizable oils include mineral oil, straight chained or branched saturated oils, and the like, especially those known for use in cosmetic applications.

Preferably, the volume ratio of the oil phase and aqueous phase in the oil-in-water emulsions of the present invention is between 1:100 and 9:1, e.g. 1:90, 1:80, 1:70, 1:60, 1:50, 1:40, 1:30, 1:20, 1:10, 1:1, 2:1, 3:1, 4:1, 5:1, 6:1, 7:1 or 8:1, more preferably between 1:50 to 1:2.

The particles used in the emulsions of the present invention are preferably amphiphilic and can be stably dispersed in the emulsion such that they adsorb to the oil-water interface to stabilize the emulsion. As mentioned above, such particles can replace surfactants in the preparation of emulsions, consequently avoiding the adverse effects of surfactants on downstream applications.

The present invention is based on the inventors' surprising finding that protein cages, owing to their intrinsic properties, such as oil-water amphiphilic properties, biocompatibility, biodegradability, homogeneous size and shape, ease of manipulation by rational design/genetic engineering, and possibility to scale-up in large quantities, can be used to overcome some of the limitations found in the known particles for use as Pickering emulsifiers. Accordingly, the emulsion of the present invention comprises at least one type of protein cages. The term "protein cage" as used herein refers to any cage-like structure with a constrained interior cavity assembled, preferably self-assembled, with precision from a predetermined number of subunits of any proteinaceous material. In particular, the protein cage of the present invention should be interpreted broadly to include all such protein cage-like structures of any size or dimension.

According to the invention the protein cage is selected from the group consisting of *Bacillus stearothermophilus* E2 protein of pyruvate dehydrogenase multi-enzyme complex (E2) having the amino acid sequence of SEQ ID NO:1, E2LC2 protein having the amino acid sequence of SEQ ID NO 2.

In certain embodiments, the protein cage is composed of protein units of *Bacillus stearothermophilus* E2 protein of pyruvate dehydrogenase multi-enzyme complex (E2) having the amino acid sequence of SEQ ID NO:1 or E2LC2 protein having the amino acid sequence of SEQ ID NO:2, and the oil is rosemary oil.

The E2 protein is part of the multienzyme pyruvate dehydrogenase complex. The pyruvate dehydrogenase complex comprises three subunits, E1, E2, and E3. In this complex, the E2 subunits form the core upon which E1 and E3 are bound. Sixty E2 subunits self-assemble into a dodecahedron cage. Its crystallographic structure shows that the assembled structure has a hollow core of approximately 25 nm in diameter with 12 openings of 5 nm each. By replacing two amino acids on wild-type E2 (E2-WT) (SEQ ID NO:1, PDB ID: 1B5S, amino acid #381-382), namely aspartic acid and glycine, by cysteine, the inventors of the present invention have created protein cage E2LC2 (SEQ ID NO: 1) with reactive -SH groups, which can be reactive with bifunctional agents, such as maleimide to attach agents to the protein cage.

The term "variant", as used herein, refers to polymorphisms, i.e. the exchange, deletion, or insertion of one or more amino acids compared to the respectively indicated amino acid sequences. Particularly, protein homologues, i.e. those having at least 30%, 40%, 50%, 60%, 70%, 80%, 90%, 95%, or 99% sequence similarity to the respectively indicated amino acid sequences, as determined by the BLAST algorithm, are also encompassed in this concept. The term "analogue" may be used interchangeably with the term "mimetic", and refers to any synthetic structures which may or may not contain amino acids and/or peptide bonds but retain the structural and functional features of a protein cage polypeptide. The term "derivative", as used herein, refers to any entities modified by genetic, physical, chemical or biochemical means.

Preferably, the protein cages may be selected to have an appropriate size, oil-water amphipathy and concentration in the emulsion in order for them to stabilize the Pickering emulsion. In an embodiment, the protein cages may be present in an amount which exceeds the amount required to stabilize the emulsion. Other factors contributing to the stability of the emulsion may include the pH, temperature, and presence of ions in the water phase as well as the presence of any other emulsifiers. The interactions of the protein cages with each other are also important. Accordingly, different kinds of protein cages may be selected to stabilize the emulsion depending on the type of emulsion (oil-in-water, water-in-oil) desired.

While the suitable amount of the protein cages for use in preparing Pickering emulsions should be determined according to the properties of the oil phase, aqueous phase, and the ratio of the oil phase to the aqueous phase, the mass fraction thereof within a Pickering emulsion is preferably within the range of 0.01 - 10 wt%.

The Pickering emulsions of the present invention may be prepared using various techniques known to those skilled in the arte.

According to non-limiting embodiments, the Pickering emulsion of the present invention may be prepared by first dispersing the protein cages in the aqueous phase, and then mixing the oil phase and the aqueous phase. The protein cages may be dispersed by vibration, stirring, ultrasonic dispersion and the like, to achieve better dispersion thereof in the aqueous phase. In general, as long as sufficient dispersion of protein cages in the aqueous phase is achieved, the dispersion mode has no significant effect on the properties of the emulsion. Suitable dispersion modes and specific operating parameters may be chosen according to the properties of the aqueous phase and particles. The mixing of the oil phase and the aqueous phase may be achieved by microfluidization, homogenization, ultrasound, two-syringe emulsification, spraying, microjet, microchannel emulsification, membrane emulsification, stirring, vibration, inversion or shaking. According to different requirements, the preferred dispersion modes include, but are not limited to microfluidization, microchannel emulsification and membrane emulsification to obtain an emulsion having a homogeneous particle size distribution. Microjet, two-syringe emulsification, homogenization, stirring or vibration may preferably be used for large-scale preparation. Different modes of mixing the oil and aqueous phase may affect the emulsion stability.

In various embodiments, the aqueous phase comprises a first agent.

In various embodiments, the oil phase comprises a second agent.

In various embodiments, the protein cage is coupled to or loaded with a third agent.

The first, second, and third agents as described herein may independently be present or absent in the Pickering emulsions of the invention, may or may not be the same agent, and may independently be selected from a protein (different from those forming the protein cage), nucleic acid molecule, organic compound, such as small molecules and/or polymers, inorganic compound, or any other molecule having the desired properties and being suited for use according to the present invention.

In various embodiments, the first, second, or third agent is a therapeutic agent, a nutritional or nutraceutical agent, or a cosmetic ingredient.

The term "therapeutic agent" as used herein refers to any agent that can elicit a therapeutic effect in a subject, i.e. ameliorates or cures a disease, disorder or condition. The term "nutritional agent" as used herein refers to components containing energy, such as fat, carbohydrates and/or proteins. The term "nutraceutical agent" as used herein refers to a substance intended to supplement a diet and provide nutrients, such as, for example, vitamins, minerals, fiber, fatty acids, or amino acids, that may be missing or may not be consumed in sufficient quantity in the diet. The term "cosmetic ingredient" as used herein includes, but is not limited to, colorants, fragrances, deodorizers, exfoliants, humectants, skin lightening agents, waterproofing agents, skin conditioning agents, anti-aging agents, or mixtures thereof. In addition to the aforementioned, the agent can also comprise auxiliaries, such as preservatives, pH adjusters, antioxidants, chelating agents, and absorbents. These may be used individually or in combination with each other or cosmetic or nutritional or therapeutic agents, as defined above.

In some embodiments, the agent can be directly or indirectly coupled to the interior or exterior surface of the protein cage through covalent, electrostatic and/or hydrophobic interactions. In some embodiments, the surface of the protein cages is modified by functional groups that can be used to allow coupling of specific agents.

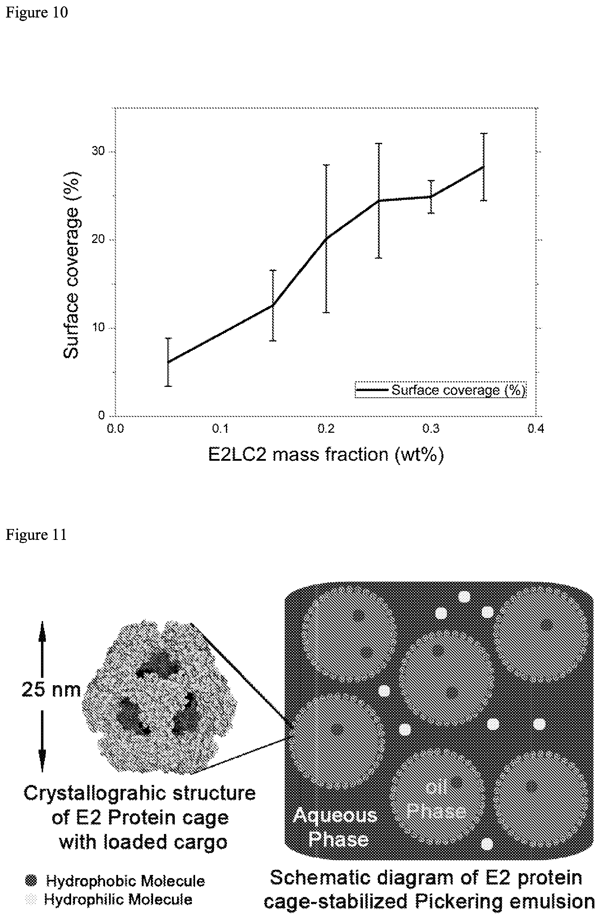

In accordance with some non-limiting embodiments of the present invention, any one, two, or three of the protein cages, the aqueous phase, and the oil phase of the Pickering emulsions may each independently contain an active agent. These active agents contained in different components may or may not be the same agent. This allows for coordinated or independent release of active agents from the different components of the Pickering emulsions. By incorporation of active agents into the protein cages, the aqueous phase, and/or the oil phase, it is possible to achieve controlled delivery of segregated active agents from a single emulsion potentially over different time scales. Similarly, where only protein cages comprise payload agents, the Pickering emulsions of the invention may also function as controlled release delivery systems. Illustrated in Figure 11 is a non-limiting embodiment of the invention wherein the aqueous phase contains a hydrophilic agent and the oil phase contains a hydrophobic agent. The protein cages thereof may or may not be coupled to or loaded with a hydrophilic or hydrophobic agent.

The term "controlled release" as used herein includes any type of controlled release including but not limited to extended release, sustained release, modified release, triggered release, delayed release, or pulsatile release.

Accordingly, the protein cages of the present invention may include a mechanism by which the release of the agent from the protein cage is controlled. Control of the release may be accomplished by controlling the opening and/or closing of the pores present in the protein cage or the integrity of the protein cage architecture itself. In one embodiment, the integrity of the protein cage architecture may be formulated such that the cage is sensitive to modification by various enzymes or other molecular cues such as the change of pH. The enzyme may be a hydrolase, the hydrolase preferably being a carbohydrase, lipase, or protease, for example, cathepsin or caspase.

In some embodiments, the protein cages include cleavable linkers for the release of the agent. For example, the present invention may provide a protein cage with a small molecule, the release of which is pH dependent. In one embodiment, the linker may be an acid labile linker, such as a hydrazone linkage. In another embodiment, a cleavable linker is incorporated into the small molecule covalently attached to the protein cage interior (See Flenniken, M. L. et al., 2005. Chemical Comm.:447-449; Willner, D., et al., 1993. Bioconjug Chem 4:521-7). Other examples of acid labile linkers include linkers formed by using cis-aconitic acid, cis-carboxylic alkatriene, polymaleic anhydride, and other acid labile linkers, such as those linkers described in U.S. Pat. Nos. 5,563,250 and 5,505,931.

In one embodiment, the linker is a photo-labile linker. Examples of photo-labile linkers include those linkers described in U.S. Pat. Nos. 5,767,288 and 4,469,774.

The agent comprised in the aqueous or oil phase of the Pickering emulsion may also be released in a controlled manner by means of any techniques known in the art. The choice of such techniques is within the knowledge of the person of average skill in the art.

In a second aspect, the present invention relates to the presently disclosed Pickering emulsions for use in pharmaceutical, cosmetic, or food applications, e.g. wherein the Pickering emulsion is used as a controlled delivery system.

The presently disclosed protein cage-stabilized Pickering emulsions may find applications in pharmaceuticals, cosmetic and food industries in that they are, for example, used for delivering active agents encapsulated within the emulsion system, for example, in the cages, in the oil, or in the water phases.

In pharmaceutical/cosmetic/food industries, according to non-limiting embodiments, combinatorial delivery systems can be designed by encapsulating hydrophilic or hydrophobic molecules in protein cages and hydrophobic molecules in the oil phase of the emulsions. The emulsions of the present invention may be used as drug delivery systems/carriers or sustained/controlled-release systems/carriers.

In food industries, according to non-limiting embodiments, Nutraceuticals can be designed based on protein cage-stabilized Pickering emulsions. These nutraceuticals can contain functional molecules including micronutrients, macronutrients, or bioactive molecules encapsulated within the emulsion system, as described herein, for delivery to specific sites under specific conditions.

In a final aspect, the present invention relates to use of the protein cages disclosed herein as emulsifiers for Pickering emulsions.

The present invention is further illustrated by the following examples. However, it should be understood, that the invention is not limited to the exemplified embodiments.

**EXAMPLES**

*Materials*

Rosemary oil, Kosher (FCC), was purchased from SAFC, Sigma-Aldrich. Mili-Q water, purified using Mili-Q Synthesis A10, Merck, was used as formulation components of Pickering emulsion. The HLB value of surfactant required to stabilized o/w emulsion with minimum droplet size using rosemary oil as an oil phase is reported as 15 and the refractive index of rosemary oil is reported as 1.468 (Rodríguez-Rojo, et al., Industrial Crops and Products 2012, 37 (1), 137-140). Alexaflour488 C5-Malemide and Nile red were purchased from Life Technologies and Sigma-Aldrich respectively.

*E2LC2 production, purification and characterization*

pET-11a vector containing E2LC2 gene (pE2LC2) was constructed by site-directed mutagenesis from pE2. E2LC2 was produced recombinantly and purified following a protocol described by Dalmau et al (Dalmau, et al., Biotechnology and Bioengineering 2008, 101 (4), 654-664). Concentration of E2LC2 was determined using BCA Protein Assay Kit (Pierce) using bovine serum albumin (BSA) as a standard. An SDS-PAGE analysis was done by running the sample on 4-20%Tris-HCl gels. The sizes of the purified E2LC2 assemblies were determined by measuring 1 mg/mL of the protein sample in buffer (20 mM Tris pH 8.7, 5mM EDTA, 0.02% sodium azide) using dynamic light scattering (DLS) with a Zetasizer Nano ZS instrument (Malvern). The correct assembly and symmetry of the protein complex was further confirmed with transmission electron microscopy (TEM) (JEOL JEM-1400). Protein samples (0.05 mg/mL) were negatively stained for 2 min with 1.5% uranyl acetate on carbon-coated electron microscopy grids (Formvar carbon film on 300 mesh copper grids, Electron Microscopy Science) and images were obtained with transmission electron microscope operating at 100 kV. Both sizes and zeta potentials of E2LC2 solution were measured under various pH conditions to determine the iso-electric point. Both measurements were done by using DLS technique using disposable capillary cell DTS1070 (Malvern).

*Microstructure of aggregation of E2LC2 at liquid-liquid interface*

The microstructure of liquid-liquid interface was examined using a confocal laser scanning microscope (Zeiss LSM 710 META) system with a 63mm oil immersion objective lens where Alexafluor488, conjugated with E2LC2, was used to aid visualization (Argon ion laser with excitation at 488nm). Samples were placed on a confocal microscope slide (25.4 × 76.2 mm; 1-1.2mm thick; Sail Brand) covered with a cover slip (24×50 mm; VFM Coverslips, Mochdre Enterprise Park), and examined with a 63× magnification lens.

*Formulation of Pickering emulsion*

Pickering emulsion was formulated by mixing rosemary oil and protein in buffer (20 mM Tris pH 8.7, 5mM EDTA, and 0.02% sodium azide) of different volume fraction by ultrasonication (Hsu, et al., J Colloid Interface Sci 2003, 259 (2), 374-81; Ghosh, et al., Journal of nanoscience and nanotechnology 2013, 13 (1), 114-22; Maa, et al., Pharmaceutical Development and Technology 1999, 4 (2), 233-240; Mirhosseini, et al., Food Hydrocolloids 2009, 23 (2), 271-280). Initially coarse emulsion was prepared in a 15 ml clear screw top vial (Sigma-Aldrich) on a magnetic stirrer by adding E2LC2 in buffer solution to the oil drop by drop using pipette. Subsequently, the coarse emulsion was subjected to ultrasonic emulsification using Vibracell cell disrupter (Model VC505, power 500 Watts and frequency 20 kHz) with maximum power output of 500 W for 2 min at 40% amplitude. Energy input was given through stepped micro tip 1/8" (630-0422, Sonics & Materials Inc.) containing a piezoelectric crystal with a probe diameter of 3 mm. The microtip was symmetrically dipped into coarse emulsion. The high pressure from the collapse of bubbles due to cavitation radiates shock wave to disturb the liquid in the neighborhood of sonicating tip, which causes the breaking up of droplets and converting the coarse emulsion to micro or nanoemulsion.

*Determination of type of emulsion*

The emulsion type (oil-in-water or water-in-oil) of formulated Pickering emulsions were determined qualitatively by drop dilution test (Hsu, et al., J Colloid Interface Sci 2003, 259 (2), 374-81). In the test, miscibility of the Pickering emulsion with pure water and rosemary oil phase were checked qualitatively. A drop of freshly formulated emulsion was mixed with a drop of mili-Q water and rosemary oil separately on a microscopic slide. Dilution in either phase will confirm about the continuous phase of emulsion. Emulsion type was also confirmed by microstructure analysis by confocal microscopy.

*Optimization of composition of formulation E2LC2-based Pickering emulsion*

Oil/water ratio, amount or mass fractions of E2LC2 as emulsifier are the most important factors that affect the formation and stability of Pickering emulsion. Optimizing these factors is the primary requirement for Pickering emulsion formulation and emulsion-based product development. In the current work, the minimum required E2LC2 amount to obtain stabile Pickering emulsion is determined by varying the compositions of rosemary oil, water, and the mass fraction of the E2LC2 (Table 1). The range of oil/water ratio (v/v) varied from 0.11 to 0.67 and the mass fraction of E2LC2 used as emulsifier to stabilize emulsion from 0.05 to 0.35 (wt%). Emulsion stability index and dispersed phase droplet size were measured to determine the optimal composition of E2LC2-stailized rosemary oil-water Pickering emulsion.

Table 1: Compositions of formulated Pickering emulsions of different rosemary oil/water ratio (v/v) and E2LC2 mass fraction (wt %)

| Set No. | Oil/ $H_2O$ ratio (v/v) | Protein mass % (wt %) | Set No. | Oil/ $H_2O$ ratio (v/v) | Protein mass % (wt %) | Set No. | Oil/ $H_2O$ ratio (v/v) | Protein mass % (wt %) |
|---|---|---|---|---|---|---|---|---|
| 01 | | 0.05 | 13 | | 0.05 | 25 | | 0.05 |
| 02 | | 0.15 | 14 | | 0.15 | 26 | | 0.15 |
| 03 | | 0.20 | 15 | | 0.20 | 27 | | 0.20 |
| 04 | 0.11 | 0.25 | 16 | 0.25 | 0.25 | 28 | 0.43 | 0.25 |
| 05 | | 0.30 | 17 | | 0.30 | 29 | | 0.30 |
| 06 | | 0.35 | 18 | | 0.35 | 30 | | 0.35 |
| 07 | 0.18 | 0.05 | 19 | 0.33 | 0.05 | 31 | 0.67 | 0.05 |
| 08 | | 0.15 | 20 | | 0.15 | 32 | | 0.15 |
| 09 | | 0.20 | 21 | | 0.20 | 33 | | 0.20 |
| 10 | | 0.25 | 22 | | 0.25 | 34 | | 0.25 |
| 11 | | 0.30 | 23 | | 0.30 | 35 | | 0.30 |
| 12 | | 0.35 | 24 | | 0.35 | 36 | | 0.35 |

### Determination of emulsion stability index (ESI)

Emulsion kinetic stability study (Cano-Medina, et al., Food Research International 2011, 44 (3), 684-692) was carried out by measuring the extent of gravitational phase separation. According to Stokes law, cream phase will observe at a certain age of emulsion of a certain droplet size because of the balance of drag force and gravitational force. For the measurement of physical stability, emulsions were formulated and stored in 15 ml clear screw top vial at room temperature for 10 days. Emulsion stability index was calculated by measuring the height of emulsion phase with respect to the total height of sample. Emulsion stability index was calculated by the equation,

$$\text{Emulsion Stability Index (ESI)} = \frac{H_E}{H_T} \times 100\%$$

$H_E$ =Height of emulsion phase; $H_T$ = Total height of sample. The measurement was performed in triplicate and average value is taken as final value.

### Measurement of dispersed phase droplet size

Dispersed phase droplet size of emulsion was measured using Dynamic Light Scattering (DLS) with a Zetasizer Nano ZS instrument using disposable cuvettes. Freshly formulated emulsions were too turbid to measure using Dynamic Light Scattering technique because highly turbid solution increases the probability of multiple scattering which can misguide the measurements. The emulsions were diluted in 100 times with mili-Q double distilled water to make it less turbid and furthermore to trim down the probability of multiple scattering before loading into zetasizer. The dilution factor was confirmed previously by measuring the size of same sample at different dilution which showed the consistency of measurement over wide range of dilution. All measurements were done in triplicates and reported values represent the average of all triplicates.

### Measurement of surface coverage

The total mean interfacial area ($S_{oil}$) in a given volume of emulsion was evaluated from the average droplet size and volume of oil emulsified as follows

$$S_{oil} = S_o = Surface\ area\ per\ droplet\ \times\ Number\ of\ droplets = S_d\ \times \frac{M_o}{V_d \times \rho_o}.$$

wherein $S_{droplet}$ is the surface area of one droplet considering the diameter of the dispersed phase oil droplet, $M_{oil}$ is the mass of emulsified oil and $V_{droplet}$ is the volume of the droplet considering the considering the diameter of the oil droplet. $\rho$ is the density of the oil at room temperature which is 903 g/dm$^3$ for rosemary oil.

The total area that protein cages, $S_{protein}$ can cover was estimated from the mass of protein cage used in formulation of emulsion as follows

$$S_{protein} = S_p = \frac{No.of\ nanocages \times Surface\ area\ can\ be\ covered\ by\ a\ single\ nanocges)}{HCP\ packing\ factor} =$$

$$\frac{\left[\frac{m_p \times N_A}{60 \times M_p} \times (\pi \times D_p^2)\right]}{0.907}.$$

wherein $m_{protein}$ is the mass of protein cage added for formulation of emulsion. Protein cages are modeled as hard spheres adsorbing onto a planner surface of rosemary oil droplet. For the percentage of surface coverage estimation, proteins were assumed to adsorb in monolayer and not to overlap. This estimation does not take into account factors such as packing efficiency of protein cage upon adsorption.

Percentage of surface coverage is calculated as,

$$Surface\ coverage = \frac{S_{protein}}{S_{oil}} \times 100\%.$$

### pH switchability of E2LC2 stabilized Pickering emulsion Characterization of emulsion

pH switchable E2LC2-stabilised Pickering emulsion was prepared by switching pH of freshly formulated emulsion from pH 8 to pH 4 by adding 1M HCl. Subsequently, the pH was restored at initial value by adding 1M NaOH. This cycle was repeated for 6 times. After each cycle droplet size of dispersed phase was measured by DLS method.

Results of this experiment leads to perform a details characterization of E2LC2 stabilized Pickering emulsion at different pH-s, ionic concentrations. Characterizations are further extended to investigate the effect of storage temperature on emulsion stability.

### Study of effect of pH

In order to investigate the effect of pH on the stability of the E2LC2-stabilised Pickering emulsion we adjust the pH of freshly formulated Pickering emulsion by adding 1M HCl or 1M NaOH stock solution. A series of the Pickering emulsion was formulated of different pH-s varied from $\approx$ 2 to 11. Droplet size and zeta potential of dispersed phase of the freshly formulated emulsion were measured within an hour of preparation.

### Study of effect of ionic concentration

A wide range of emulsions were prepared at different ionic strengths from 10mM to 500mM to investigate the effect of ionic concentration on the stability of the E2LC2-based Pickering emulsion. 1M NaCl was added to freshly prepared emulsion to obtain the desired final ionic concentration. The final volume of emulsion was maintained equal by adding mili-Q water to the emulsion as water is continuous phase in emulsion. Zeta potential of freshly prepared Pickering emulsions was measured at day 2 and day 10 and droplet size of dispersed phase was measured at day 2, 6 and 10 from formulation which was stored at room temperature.

### Study of storage temperature

To investigate the applicability of E2LC2-stabilized Pickering emulsion, the freshly formulated emulsions were stored at different temperatures like room temperature, 25°C; human body temperature, 37°C and elevated temperature, 50°C. Freshly formulated Pickering emulsions were stored at room temperature, 37°C incubator and 50°C incubator for maintaining temperatures. The stability of the emulsions was determined by measuring the disperse phase zeta potential and droplet size. Zeta potentials of the emulsions were measured within an hour of preparation and at day 10 from formulation. Droplet sizes of the emulsions were measured at day 2, 6 and 10 from formulation.

*Measurement of zeta potential ($\zeta$)*

The zeta potential of the emulsion droplets were measured with the Zetasizer Nano ZS (Malvern, Westborough, MA) using disposable capillary cell DTS1070 (Malvern, Westborough, MA). Sample preparation technique was similar to the preparation technique used in droplet size measurement. The sample was loaded into capillary cell using a 1ml syringe after inverting the cell to ensure the avoidance of tiny air bubble into the cell. The sample was analyzed at a scattering angle 173° and the effective electric field applied in the capillary cell was 150V. The surface charge properties of the droplets were investigated by zeta potential measurements as a function of pH, ionic strength, and storage age and storage temperatures. At least two separate measurements were performed for each sample.

*Formulation and rheological analysis of emulsion gel-like system*

A rheological analysis was performed to compare the rheological behavior of E2LC2 stabilized Pickering emulsion formulated with two different oil/water ratio, 0.11 and 0.66. Two different rheological measurements were made in order to characterize the emulsions. First, shear stress versus shear rate runs were applied to the emulsion samples over a shear rate range of 0.01-500 s$^{-1}$. Secondly, oscillatory rheological measurements were made in the linear viscoelastic region to measure the storage or elastic modulus (G'), the loss or viscous modulus (G"), and the loss tangent (tan $\delta$) by oscillatory frequency sweep tests which was carried out between 0.1 to 100 rad/sec angular frequency at constant strain of 1%. Rheological measurements were performed in a controlled stress rheometer AR2000 (TA Instruments, Delaware, USA) fitted with a parallel plate geometry (25 mm diameter, gap 1000 um). A Peltier system in the bottom plate provided fast and accurate temperature control.

**Example 1: Characterization of the purified E2LC2**

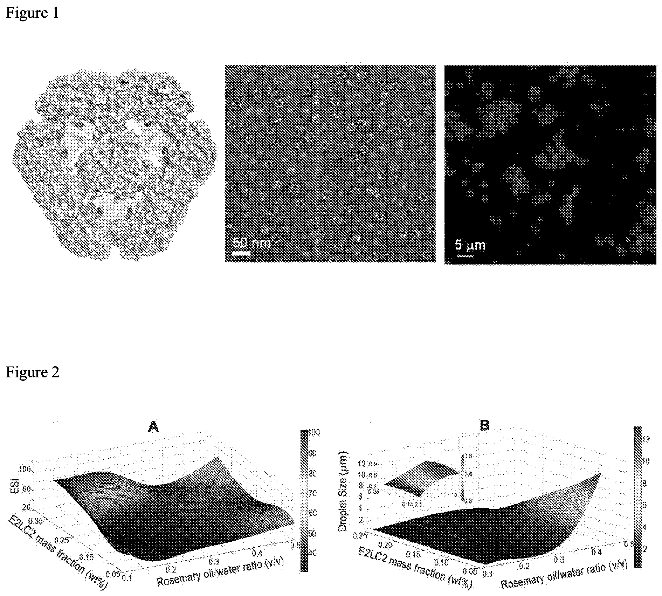

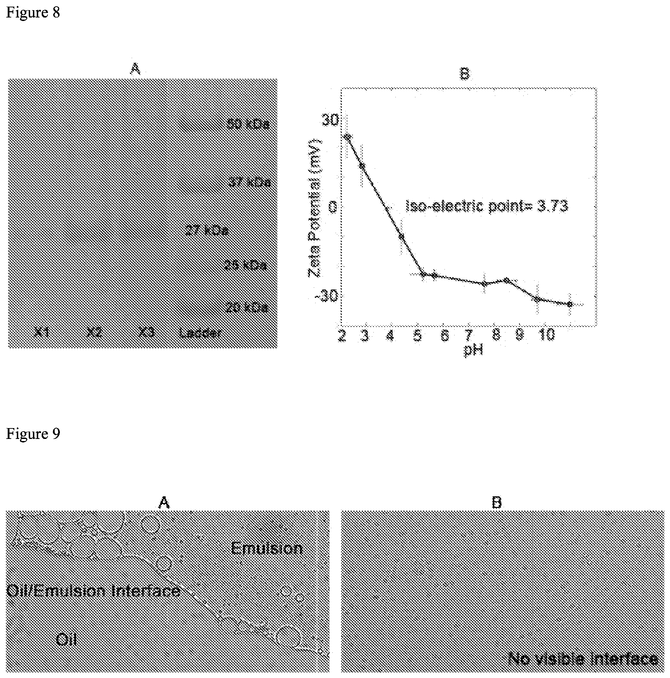

The purity of E2LC2 was confirmed by performing SDS-PAGE analysis (Figure 8). The theoretical molecular weight of E2LC2 is 26.426 kDa (calculated using Expasy Protparam tool). A single band at around 27 kDa on SDS-PAGE confirmed the purity of E2LC2 obtained from the flow-through fraction of ion exchange chromatography (IEX). The hydrodynamic diameter of E2LC2 was measured to be 25 nm with polydispersity index of <0.2, which was consistent with previously published data for E2-WT and indicated that the purified E2LC2 was monodispersed (Milne, et al., Molecular architecture and mechanism of an icosahedral pyruvate dehydrogenase complex: a multifunctional catalytic machine. 2002; Vol. 21, p 5587-5598). This observation suggestd that the E2LC2 properly assembles into caged structure similar to the E2-WT. The assembly and the dodecahedron hollow cage shape of E2LC2 was further confirmed by transmission electron microscopy (TEM) as shown in Figure 1B. The isoelectric point for E2LC2 was determined to be 3.73 by measuring its zeta potential at different pH-s (Figure 8). At pH>7, the absolute value of zeta potential was greater than 30 mV suggesting the stability of E2LC2 in Tris buffer solution.

The surface activity of E2LC2 was explored by mixing it with two immiscible liquids, water and rosemary oil. Figure 1C showed that E2LC2 adsorbed at water and rosemary oil interface and confirmed the presence of adsorbed layer of protein cages which appears to cover the surface of the oil droplets. Several studies have reported that the structure of globular protein may deform at water/oil interface (Shlomo & Alexander, Introduction. In Surface Activity of Proteins, CRC Press: 1996; pp 1-38; McClements, Current Opinion in Colloid & Interface Science 2004, 9 (5), 305-313). As E2LC2 is soluble and stable in aqueous condition and hence hydrophilic by nature, it was expected to deform and undergo conformational changes exposing the hydrophobic patches on its surface to achieve the maximum favorable interaction at the interface between of water and rosemary oil. The zeta potential of E2LC2 dispersed in 20 mM Tris buffer (pH 8.7) was about -27 mV while the zeta potential of the formulated emulsion droplets coated with E2 protein cage increased negatively to about -35 to -50 mV (depending on the formulation composition). The observations indicated that structural changes such as deformation may occur upon adsorption of E2LC2 on the water/rosemary oil interface. The adsorption of E2LC2 covered the surface of rosemary oil droplets and was shown to stabilize the water/rosemary oil system by forming a dense and continuous interfacial layer around the droplets.

**Example 2: Formulation of E2LC2-stabilized Pickering emulsions**

Pickering emulsion was formulated and its type was determined by drop dilution test (Hsu, et al., J Colloid Interface Sci 2003, 259 (2), 374-81). According to Finkle's rule (Dickinson, Journal of the Science of Food and Agriculture 2013, 93 (4), 710-721), protective barrier around dispersed phase droplet is enhanced by particles that are preferentially wetted in emulsion continuous phase. In the current work, E2LC2 was used as emulsifier and the formulated emulsions were oil-in-water emulsion (Figure 9).

**Example 3: Optimization of formulation compositions**

Emulsion composition of formulation was optimized to obtain stable Pickering emulsion by varying rosemary oil/water ratio and mass fraction of E2LC2. To determine the stability of Pickering emulsion, variation of emulsion stability index (ESI) and droplet size of dispersed phase were measured.

The effect of mass fraction of E2LC2 and the effect of rosemary oil/water ratio on the emulsion after 10 days of shelf-life at ambient condition were observed visually. Several emulsions experienced creaming effect that full separation of emulsion phase at the top and serum (or aqueous) phase at the bottom occurred. Results of this experiment are shown in a surface plot in Figure 2A in terms of ESI of Pickering emulsions of different compositions. Emulsions with oil fraction 0.1-0.2 (v/v) and E2LC2 mass fraction 0.30-0.35 (wt %) showed higher stability with maximum ESI, 100. Lowering the E2LC2 mass fraction to less than 0.3 (wt %) resulted in lower stability as shown by separated emulsion phase from the serum phase within a few days. A sharp decrease of emulsion stability occured as the rosemary oil/water ratio increased at constant E2LC2 mass fraction of 0.3 and 0.35 (wt %). The decreasing trend of stability continued until the rosemary oil/water ratio reached 0.25. Beyond rosemary oil/water ratio, 0.25, the stability of emulsions continued to increase despite higher rosemary oil/water ratio. At this particular oil-water ratio the formation of gel structure initiated and continued to stabilize the emulsion by forming network among the droplets. Formation of network between droplets in emulsion was analyzed by measuring rheological properties.

Droplet size of emulsion dispersed phase was measured at 10 days of shelf-life for all emulsions formulated for optimization. The result of droplet size profiling with different rosemary oil/water ratio and mass fraction of E2LC2 was presented in a 3D surface plot (Figure 2B). Droplet size increased as the oil/water ratio increased at lower E2LC2 mass fraction. A low supply of E2LC2 in formulation of Pickering emulsion caused high rate of coalescence of oil droplets resulting in droplets size >10 $\mu$m, while at higher E2LC2 mass fraction, coverage of surface of droplets increased prohibiting the droplets to coalesce (data of percentage of surface coverage is shown in Figure 10). As a result, at higher E2LC2 mass fraction, droplet size of Pickering emulsion remained <2 $\mu$m. Pickering emulsion with the lowest droplet size of 200-400 nm was formed at E2LC2 mass fraction 0.30-0.35 (wt %) and at lower oil/water ratio, between 0.10-0.20. In this region of surface plot, the dispersed phase oil droplet size resembled formations of nano emulsion. At higher E2LC2 mass fraction and lower oil fraction droplet, higher surface coverage was achieved resulting in lower droplet size and higher stability.

From the optimization experiment, emulsion with rosemary oil/water ratio of 0.11 and E2LC2 mass fraction of 0.35 wt% showed great stability with ESI value of 100 after 10 days of shelf-life and the droplet size was <300 nm.

**Example 4: pH switchability of E2LC2 stabilized Pickering emulsions**

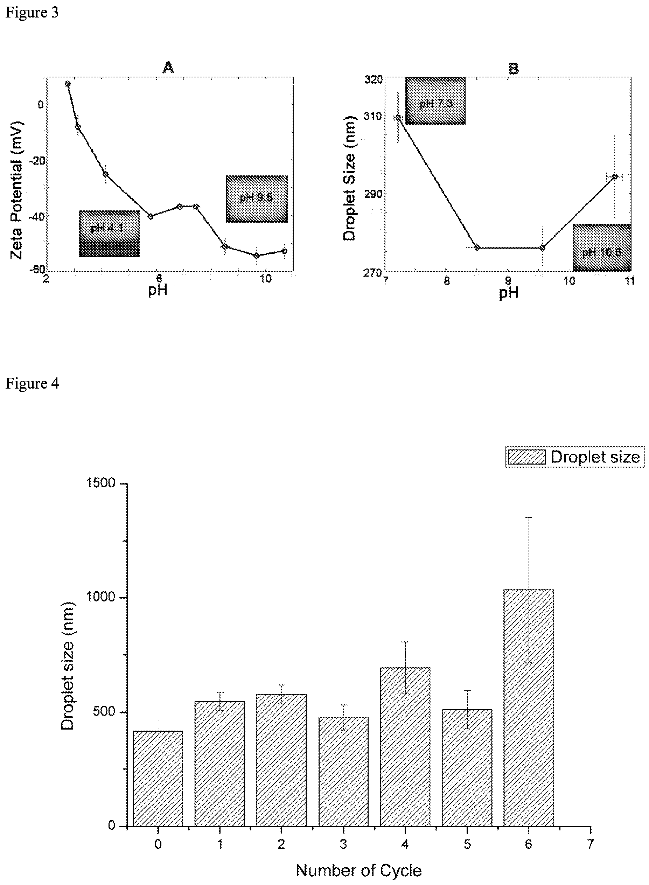

E2LC2-stabilized Pickering emulsion could be destabilized by switching the pH of freshly formulated emulsion from pH 8 to pH 4. The emulsion separated into emulsion and serum phases. Subsequent restoration to pH 8 resulted in the formation of stable emulsion with the highest ESI (Figure 4, inset). This cycle of lowering pH value and restoring the value to initial one was repeated for 7 times. The observation from each cycle up to the 6th cycle was similar, illustrating the formation of pH-switchable E2LC2-stabilized Pickering emulsion and that the process was reversible. The droplet size of the emulsion, measured after each cycle, increased. After the completion of the 6th cycle, Pickering emulsion could not be restored to its stabile state even though the pH was restored at 8. Similar observation had been reported by Shijei Ding et al. for Pickering emulsion stabilized by palygorskite particles (Lu, et al., Applied Clay Science 2014, 102 (0), 113-120), Li and Stoever (Li, et al., Langmuir 2008, 24 (23), 13237-13240) for emulsion stabilized by small organic molecule with charged group. pH responsive behavior of Pickering emulsion mostly occured at pH close to isoelectric point of emulsifier which was measured to be 3.73 for E2LC2 (Figure 8B). At iso-electric point the surface charge decreased and thereby the hydrophobicity increased. Reduction of surface charges resulted in reduction of electrostatic repulsion forces which triggered flocculation. The proposed mechanism of the irreversibility at 7th cycle was that at each pH switch cycle, the droplets may coalesce in addition to flocculation. The coalesced droplets required energy to regain its initial form of smaller droplets. Since there was no energy input during the experiment, as the cycle was repeated more droplets coalesced and the emulsion was irreversibly separated. This phenomenon also may result from the continuous increase of ionic strength of Pickering emulsion after each cycle by the addition of HCl and NaOH to tune the pH.

Following this observation, a detail characterization of the emulsion at different pH values and ionic concentrations was performed.

**Example 5: Effect of pH on the stability of Pickering emulsions**

The pH value considerably affected the stability of Pickering emulsions during the production of food or pharmaceutical products. The change of pH during processing, consumption or digestion could stabilize or destabilize Pickering emulsion. In the current work, the effect of pH on E2LC2 stabilized Pickering emulsion (rosemary oil/water ratio of 0.11 (v/v) and E2LC2 mass fraction of 0.35 (wt %)) was investigated by varying the value of pH from 2 to 11 where the other variables remained constant.

Visual observation of the emulsions at different pH-s suggested that, Pickering emulsion was very stable at higher pH-s, neutral to basic range, where at pH<4, Pickering emulsion experienced destabilization. Visual observation was further confirmed by measuring zeta potential and droplet size of freshly formulated Pickering emulsion at various pH-s. Results shown in Figure 3A indicated the instability of Pickering emulsion at lower pH value as the absolute value of zeta potential was less than 30 mV. At pH>7, absolute zeta potential value was higher than 30 mV resulting in stable emulsion. The increasing trend of absolute value of zeta potential of emulsion was similar to the increasing trend of zeta potential of E2LC2 in the Tris buffer solution (Figure 8B). Figure 3B showed that the droplet size agreed with the results of visual stability analysis and zeta potential measurements. The droplet size continued to decrease with increasing pH. However, an increase in droplet size was observed at pH>9.5, the Pickering emulsion was stable with zeta potential value >30mV. The lowest droplet size of ~300 nm was observed at pH 9.

Results of this analysis suggested that pH played an important role in stabilizing Pickering emulsion by modulating the surface charge E2LC2. pH value close to iso-electric point was not favored for emulsion stability as the surface activity of protein decreased (de Folter, et al., Soft Matter 2012, 8 (25), 6807-6815). On the other hand, at higher pH, neutral to basic range, emulsion of the lowest droplet size could be formed with the highest stability index value. Several studies on protein-based Pickering emulsion showed similar results (Tan, et al., LWT - Food Science and Technology 2014, 57 (1), 376-382).

**Example 6: Effect of ionic strength on the stability of Pickering emulsions**

It was hypothesized that the ionic strength influences the stability of E2LC2-stabilized Pickering emulsion. To test this hypothesis, stability of the emulsion of different ionic concentrations was investigated. The droplet size of dispersed rosemary oil phase as well as zeta potential were measured at different shelf life times to determine the effect of ionic concentration on stability of E2LC2 stabilized Pickering emulsion.

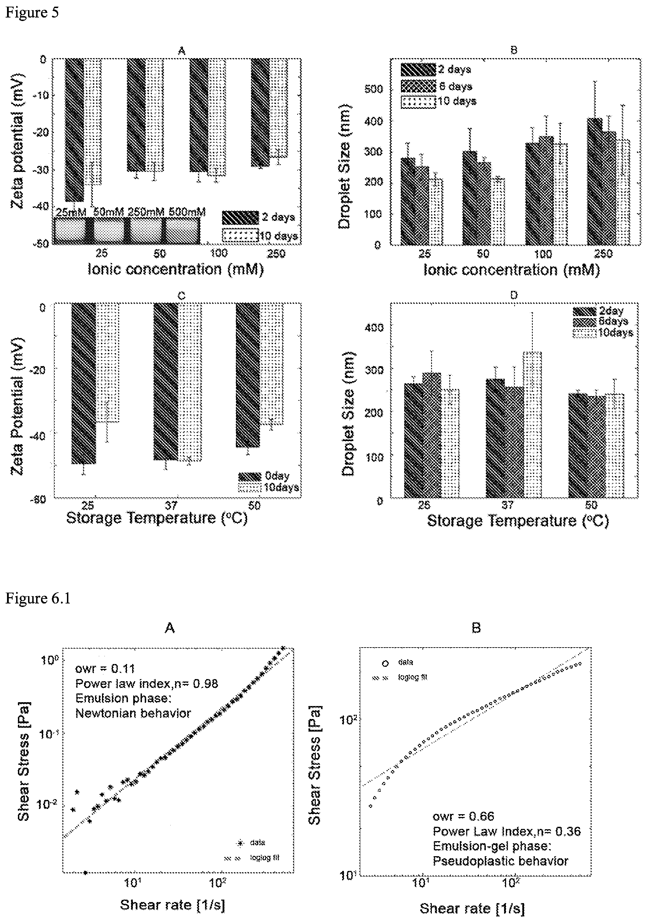

The bar plot (Figure 5A) showed a decreasing trend of zeta potential with the increase of ionic strength of Pickering emulsion. The decreasing trend of zeta potential with increasing ionic strength is a classical behavior in colloidal system. The surface charge of emulsion droplet decreases with increasing ionic strength due to the salt screening effect where the electrostatic interaction between oppositely charged species is reduced due to neutralization by counter ions. This phenomenon leads to the decrease of electrostatic double layer thickness as explained by the theory of electric double layer compression with the increase of ionic strength (Bohinc, et al., Electrochimica Acta 2001, 46 (19), 3033-3040). The characteristic thickness of double layer is called Debye length, $k^{-1}$, which is reciprocally proportional to the square root of the ion concentration. As the thickness of double layer decreases with the ionic strength, the zeta potential of the Pickering emulsion also follows similar decreasing trend. Inset in Figure 5A depicted the separation of emulsion phase from serum phase at ionic concentration of 250 mM where the emulsion with 25 mM and 50 mM showed no separation.

The dispersed phase droplet size of E2LC2-stablized Pickering emulsion was measured at age of 2, 6 and 10 days. From the bar plot (Figure 5B) the increasing trend of droplet size with ionic strength could be observed which could be explained by the decrease of zeta potential. As the zeta potential reduced with ionic strength, the electrostatic repulsion between dispersed droplets was reduced leading to increase of z-average droplet size by coalescence and destabilization of the Pickering emulsion.

**Example 7: Effect of storage temperature on the stability of Pickering emulsions**

Characterization of E2LC2-stabilized Pickering emulsion was further extended to investigate the effects of storage temperature on the stability of emulsion as emulsion-based products like food and pharmaceutical experiences different thermal conditions during production, transportation as well as consumption. In this work, the droplet size and zeta potential of dispersed phase at different storage temperatures and life times were measured.

Figure 5C showed that despite the minute reduction, the absolute value of zeta potential was greater than 30 mV across all temperatures suggesting that the Pickering emulsion was stable for 10 days up to 50°C incubation. The observation was further supported by the droplet size measurements. At 25°C and 50°C the droplet sizes remained similar. The slight increase of droplet size at 37°C may result from the minute reduction of zeta potential as well as the surface charge but was not expected to affect the stability of the emulsion.

**Example 8: Formation and characterization of emulsion gel-like network**

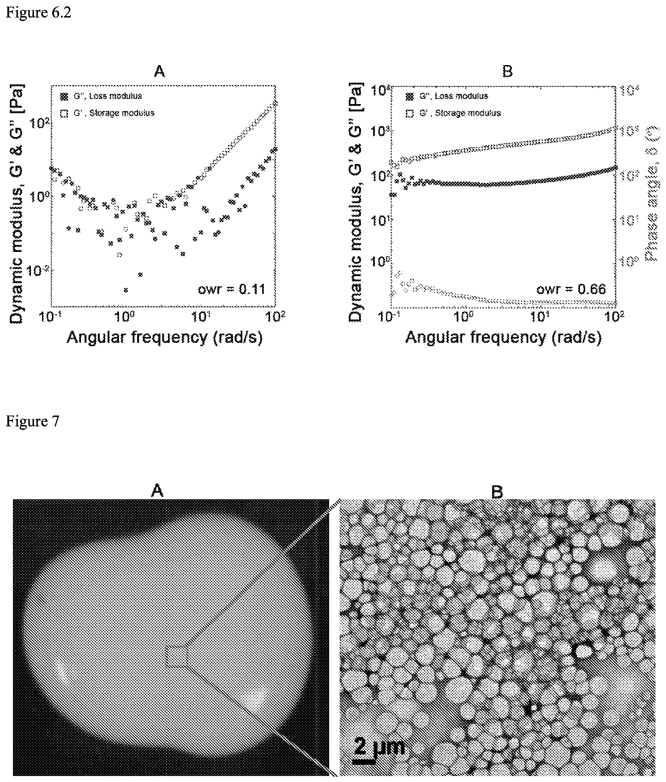

The formation of emulsion gel-like network was hypothesized during optimization of composition for emulsion formulation. The formation of emulsion gel-like network was further confirmed by comparing rheological properties of emulsions prepared with different oil/water ratio, 0.11 and 0.66. Linear viscoelastic region had been determined before conduction rheological analysis (data not shown here). Figure 6 illustrated the results of oscillatory viscometry analysis of these two emulsions.

Figure 6A illustrated the viscosity versus shear rate curve fitted with power law equation as follows:

$$\eta = k\gamma^{n-1},$$

where, $\eta$= viscosity (Pa.s), $\gamma$= shear rate (s$^{-1}$), k= consistency index and n is Power Law index. The value of n reflects the behavior of systems. If n<1, the system shows shear thinning behavior where the system becomes shear thickening if n>1 and if n=1, system shows Newtonian behavior. The value of n, power law index, calculated from viscosity versus shear rate curve for emulsion with oil/water ratio 0.11 and 0.66 are 0.98 and 0.36, respectively. Both of the emulsions showed shear thinning behavior. Shear thinning behavior in emulsion indicats the presence of weak attractive forces between the emulsion droplets, which rises to a formation of weak emulsion gel-like structure (Torres, et al., Colloids and Surfaces A: Physicochemical and Engineering Aspects 2007, 302 (1-3), 439-448). A resistance to flow provided by the network arises from weak interaction forces. If the shear stress is lower than the attractive forces between droplets, then the shear energy will store as an extension of bonds between dispersed phase droplets. This phenomenon increases the resistance of the system. As a result, system will not flow and it will show elasticity. When the stress becomes larger than the resistance forces the system started to flow. The application of stress causes the droplets to move from each other.

Oscillatory rheological measurements of elastic modulus (G') and viscous modules (G") can indicate whether the emulsion system is strongly or weakly associated. Values of phase angle shift, $\delta$ can also provide information about the nature of the viscoelastic response of the emulsion system. In elastic networks $\delta$ is 0°, whereas in purely viscous liquids $\delta$ is 90°. For viscoelastic systems $\delta$ takes some value in this range. The closer $\delta$ is to 0° the more the emulsion system displays an elastic response to the application of the shear stress and thus the more developed is the gel-like colloidal network.

The magnitude of elastic modulus (G') was higher than viscous modules (G") and both were independent of frequency of the emulsion formulated with high rosemary oil/water ratio, 0.66, which was a clear indication of network formation (Torres, et al., Colloids and Surfaces A: Physicochemical and Engineering Aspects 2007, 302 (1-3), 439-448) (Figure 6B). The phase angle value was also independent of frequency range and maintained as 8° for wide range of frequency, indicating that the emulsion gel-like system tended to show elastic response to shear. On the other hand, emulsion formulated with low rosemary oil/water ratio, 0.11, shows complex behavior of elastic and viscous modulus and values were not independent of frequency. Thus the emulsion formulated with low rosemary oil/water ratio had no or very little network between dispersed droplets.

The formation of gel-like system at high oil/water ratio was further confirmed by exploring its microstructure under transmission electron microscopy. Figure 7B showed the microstructure of emulsion gel formulated at higher oil and protein fraction. At high rosemary oil/water ratio, 0.66 (v/v), and E2LC2 mass fraction, 0.35% (wt %), flocculation of oil droplets may occur and trigger the formation of soft solid emulsion gel network. The electron micrograph showed closely packed droplets of ~2 $\mu$m.

E2LC2 is surface active nanoparticle which can stabilize a Pickering emulsion with high stability by adsorbing at the interface of two immiscible liquid phases. The optimal composition of E2LC2-stabilized Pickering emulsion was determined to be rosemary oil/water ratio 0.11 (v/v) with protein mass fraction 0.35 (wt %). The emulsion showed excellent stability in neutral to basic pH, ionic concentration up to 250 mM, and storage temperature up to 50°C. The optimized Pickering emulsion was pH switchable (from pH 4 to pH 8) and the process was reversible up to six cycles. At high rosemary oil/water ratio emulsion formed gel-like network showing viscoelastic property. This work showed that E2 protein cage functioning as Pickering emulsifier can be used in the development of protein cage-based products for applications in food, pharmaceuticals, and personal care products.

**Claims**

1. Pickering emulsion comprising an aqueous phase, an oil phase immiscible with said aqueous phase, and a nanoparticle dispersed in said aqueous phase and adsorbed to the liquid-liquid interface between said aqueous phase and said oil phase, wherein said nanoparticle is a protein cage and wherein the protein cage is composed of protein units selected from the group consisting of *Bacillus stearothermophilus* E2 protein of pyruvate dehydrogenase multi-enzyme complex (E2) having the amino acid sequence of SEQ ID NO:1, E2LC2 protein having the amino acid sequence of SEQ ID NO:2.

2. The Pickering emulsion according to claim 1, wherein the aqueous phase is any one selected from the group consisting of water and aqueous solutions.

3. The Pickering emulsion according to any one of claims 1-2, wherein the oil is selected from the group consisting of essential oils, vegetable oils, mineral oils, organic oils, lipids, and any water-immiscible liquids.

4. The Pickering emulsion according to any one of claims 1-3, wherein the aqueous phase comprises a first agent, preferably wherein the oil phase comprises a second agent.

5. The Pickering emulsion according to any one of claims 1-4, wherein the protein cage is coupled to or loaded with a third agent.

6. The Pickering emulsion according to claim 4 or 5, wherein the first, second, or third agent is selected from a protein (different from those forming the protein cage), nucleic acid molecule, organic compound, inorganic compound, or any other molecule.

7. The Pickering emulsion according to any one of claims 4-6, wherein the first, second, or third agent is a therapeutic agent, a nutritional or nutraceutical agent, or a cosmetic ingredient.

8. The Pickering emulsion according to any one of claims 4-7, wherein the Pickering emulsion is a controlled delivery system for the first, second, or third agent.

9. The Pickering emulsion according to any one of claims 1-8, wherein the Pickering emulsion is a Pickering emulsion-gel.

10. The Pickering emulsion according to any one of claims 1-9 for use in pharmaceutical, cosmetic, or food applications, wherein the Pickering emulsion is used as a controlled release delivery system.

11. Use of protein cages as emulsifiers for the Pickering emulsion according to any one of claims 1-9.


**Patentansprüche**

1. Pickering-Emulsion, umfassend eine wässrige Phase, eine mit der wässrigen Phase nicht mischbare Ölphase und ein in der wässrigen Phase dispergiertes und an der Flüssigkeits-Flüssigkeits-Grenzfläche zwischen der wässrigen Phase und der Ölphase adsorbiertes Nanopartikel, wobei das Nanopartikel ein Proteinkäfig ist und wobei der Proteinkäfig aus Proteineinheiten besteht, die aus der Gruppe ausgewählt sind, die aus dem E2-Protein von *Bacillus stearothermophilus* des Pyruvatdehydrogenase-Multienzymkomplexes (E2) mit der Aminosäuresequenz von SEQ ID NO:1 und dem E2LC2-Protein mit der Aminosäuresequenz von SEQ ID NO:2 besteht.

2. Die Pickering-Emulsion gemäß Anspruch 1, wobei die wässrige Phase eine beliebige aus der Gruppe bestehend aus Wasser und wässrigen Lösungen ist.

3. Die Pickering-Emulsion gemäß einem der Ansprüche 1-2, wobei das Öl aus der Gruppe ausgewählt ist, die aus ätherischen Ölen, Pflanzenölen, Mineralölen, organischen Ölen, Lipiden und beliebigen mit Wasser nicht mischbaren Flüssigkeiten besteht.

4. Die Pickering-Emulsion gemäß einem der Ansprüche 1-3, wobei die wässrige Phase ein erstes Mittel umfasst, vorzugsweise wobei die Ölphase ein zweites Mittel umfasst.

5. Die Pickering-Emulsion gemäß einem der Ansprüche 1 bis 4, wobei der Proteinkäfig an ein drittes Mittel gekoppelt oder mit diesem beladen ist.

6. Die Pickering-Emulsion gemäß Anspruch 4 oder 5, wobei das erste, zweite oder dritte Mittel aus einem Protein (das sich von denen unterscheidet, die den Proteinkäfig bilden), einem Nukleinsäuremolekül, einer organischen Verbindung, einer anorganischen Verbindung oder einem anderen Molekül ausgewählt ist.

7. Die Pickering-Emulsion gemäß einem der Ansprüche 4 bis 6, wobei das erste, zweite oder dritte Mittel ein therapeutisches Mittel, ein Nahrungs- oder Nutrazeutikum oder ein kosmetischer Inhaltsstoff ist.

8. Die Pickering-Emulsion gemäß einem der Ansprüche 4 bis 7, wobei die Pickering-Emulsion ein kontrolliertes Freisetzungssystem für das erste, zweite oder dritte Mittel ist.

9. Die Pickering-Emulsion gemäß einem der Ansprüche 1 bis 8, wobei die Pickering-Emulsion ein Pickering-Emulsionsgel ist.

10. Die Pickering-Emulsion gemäß einem der Ansprüche 1 bis 9 zur Verwendung in pharmazeutischen, kosmetischen oder Lebensmittelanwendungen, wobei die Pickering-Emulsion als kontrolliertes Fresetzungssystem verwendet wird.

11. Verwendung von Proteinkäfigen als Emulgatoren für die Pickering-Emulsion gemäß einem der Ansprüche 1 bis 9.

**Revendications**

1. Émulsion de Pickering comprenant une phase aqueuse, une phase huileuse non miscible avec ladite phase aqueuse, et une nanoparticule dispersée dans ladite phase aqueuse et adsorbée à l'interface liquide-liquide entre ladite phase aqueuse et ladite phase huileuse, dans laquelle ladite nanoparticule est une cage protéique et dans laquelle la cage protéique est composée d'unités protéiques choisies dans le groupe constitué par la protéine E2 de *Bacillus stearothermophilus* du complexe multi-enzymatique pyruvate déshydrogénase (E2) ayant la séquence d'acides aminés de SEQ ID NO:1, la protéine E2LC2 ayant la séquence d'acides aminés de SEQ ID NO:2.

2. L'émulsion de Pickering selon la revendication 1, dans laquelle la phase aqueuse est choisie parmi le groupe constitué d'eau et de solutions aqueuses.

3. Émulsion de Pickering selon l'une quelconque des revendications 1 à 2, dans laquelle l'huile est choisie parmi le groupe constitué d'huiles essentielles, d'huiles végétales, d'huiles minérales, d'huiles organiques, de lipides et de tout liquide non miscible à l'eau.

4. Émulsion de Pickering selon l'une quelconque des revendications 1 à 3, dans laquelle la phase aqueuse comprend un premier agent, de préférence dans laquelle la phase huileuse comprend un deuxième agent.

5. L'émulsion de Pickering selon l'une quelconque des revendications 1 à 4, dans laquelle la cage protéique est couplée à ou chargée avec un troisième agent.

6. L'émulsion de Pickering selon la revendication 4 ou 5, dans laquelle le premier, le deuxième ou le troisième agent est choisi parmi une protéine (différente de celles formant la cage protéique), une molécule d'acide nucléique, un composé organique, un composé inorganique ou toute autre molécule.

7. Émulsion de Pickering selon l'une quelconque des revendications 4 à 6, dans laquelle le premier, le deuxième ou le troisième agent est un agent thérapeutique, un agent nutritionnel ou nutraceutique, ou un ingrédient cosmétique.

8. Émulsion de Pickering selon l'une quelconque des revendications 4 à 7, dans laquelle l'émulsion de Pickering est un système de libération contrôlée pour le premier, le deuxième ou le troisième agent.

9. Émulsion de Pickering selon l'une quelconque des revendications 1 à 8, dans laquelle l'émulsion de Pickering est un gel d'émulsion de Pickering.

10. Émulsion de Pickering selon l'une quelconque des revendications 1 à 9, destinée à être utilisée dans des applications pharmaceutiques, cosmétiques ou alimentaires, dans laquelle l'émulsion de Pickering est utilisée comme système de libération contrôlée.

11. Utilisation de cages protéiques comme émulsifiants pour l'émulsion de Pickering selon l'une quelconque des revendications 1 à 9.

Figure 1

Figure 2

Figure 3

Figure 4

Figure 5

Figure 6.1

Figure 6.2

Figure 7

Figure 8

Figure 9

Figure 10

Figure 11

25 nm

Crystallograhic structure
of E2 Protein cage
with loaded cargo

● Hydrophobic Molecule
　Hydrophilic Molecule

Schematic diagram of E2 protein
cage-stabilized Pickering emulsion

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- SG 10201600290W
- US 5563250 A
- US 5505931 A
- US 5767288 A
- US 4469774 A

### Non-patent literature cited in the description

- **PICKERING**. Journal of the Chemical Society. *Transactions*, 1907, vol. 91 (0), 2001-2021
- **RAMSDEN**. *Proceedings of the Royal Society of London*, 1903, vol. 72 (477-486), 156-164
- **FUJII S. et al.** *Journal of Colloid and Interface Science*, 2009, vol. 388 (1), 222-228
- **FLENNIKEN, M. L. et al.** *Chemical Comm.*, 2005, 447-449
- **WILLNER, D. et al.** *Bioconjug Chem*, 1993, vol. 4, 521-7
- **RODRÍGUEZ-ROJO et al.** *Industrial Crops and Products*, 2012, vol. 37 (1), 137-140
- **DALMAU et al.** *Biotechnology and Bioengineering*, 2008, vol. 101 (4), 654-664
- **HSU et al.** *J Colloid Interface Sci*, 2003, vol. 259 (2), 374-81
- **GHOSH et al.** *Journal of nanoscience and nanotechnology*, 2013, vol. 13 (1), 114-22
- **MAA et al.** *Pharmaceutical Development and Technology*, 1999, vol. 4 (2), 233-240
- **MIRHOSSEINI et al.** *Food Hydrocolloids*, 2009, vol. 23 (2), 271-280
- **CANO-MEDINA et al.** *Food Research International*, 2011, vol. 44 (3), 684-692
- **MILNE et al.** *Molecular architecture and mechanism of an icosahedral pyruvate dehydrogenase complex: a multifunctional catalytic machine.*, 2002, vol. 21, 5587-5598
- **SHLOMO ; ALEXANDER**. Surface Activity of Proteins. CRC Press, 1996, 1-38
- **MCCLEMENTS**. *Current Opinion in Colloid & Interface Science*, 2004, vol. 9 (5), 305-313
- **DICKINSON**. *Journal of the Science of Food and Agriculture*, 2013, vol. 93 (4), 710-721
- **LU et al.** *Applied Clay Science*, 2014, vol. 102 (0), 113-120
- **LI et al.** *Langmuir*, 2008, vol. 24 (23), 13237-13240
- **DE FOLTER et al.** *Soft Matter*, 2012, vol. 8 (25), 6807-6815
- **TAN et al.** *LWT - Food Science and Technology*, 2014, vol. 57 (1), 376-382
- **BOHINC et al.** *Electrochimica Acta*, 2001, vol. 46 (19), 3033-3040
- **TORRES et al.** *Colloids and Surfaces A: Physicochemical and Engineering Aspects*, 2007, vol. 302 (1-3), 439-448